# EUROPEAN PATENT APPLICATION

(11) **EP 0 602 393 A1**
(43) Date of publication of application: **22.06.1994**
(21) Application number: 93118359.4
(22) Date of filing: 12.11.1993
(51) Int. Cl.: A61M 5/172, H05K 5/00

(54) **Interlocking module system**

(30) Priority: 13.11.1992 US 976404
(71) Applicant: MINNESOTA MINING AND MANUFACTURING COMPANY, St. Paul, Minnesota 55133-3427 (US)
(72) Inventor: Anderson, Jerald W., c/o Minnesota Mining and, St. Paul, Minnesota 55133-3427 (US); Dretzka, Philip C., c/o Minnesota Mining and, St. Paul, Minnesota 55133-3427 (US); Belden, Tighe M., c/o Minnesota Mining and, St. Paul, Minnesota 55133-3427 (US); Struble, Kent R., c/o Minnesota Mining and, St. Paul, Minnesota 55133-3427 (US); Sutherland, Daniel M., c/o Minnesota Mining and, St. Paul, Minnesota 55133-3427 (US); Toycen, Mark A., c/o Minnesota Mining and, St. Paul, Minnesota 55133-3427 (US)
(74) Representative: VOSSIUS & PARTNER

(57) **Abstract**

A system for interlocking a plurality modules (210), such as medical instrument modules (e.g., infusion pumps), in side-by-side relationship such that not more than one module (210) can be mounted on each side of a center module. The modules (210) are interchangeable in that any module of the system can be a center module or a side module. In the preferred embodiment, latching arms (216 and 218) on a module (210) are automatically moved as the module is mounted on a pole stand (224) to allow other "side" modules to be mounted on that "center" module. The latching arms (216 and 218) of the "side" modules prevent additional modules (210) from being mounted on the side modules. Other embodiments are described as well.

## Description

The invention relates generally to a system for mounting modules on one another, and more particularly to a system for mounting modules, such as medical instrument modules, on a pole stand.

In modern medical practice a variety of diagnostic and therapeutic instruments are used, sometimes to such a degree that floor and shelf space near the patient's bedside is at a premium. One known solution to the problem of mounting instrument modules is the use of a pole stand, which allows equipment to be mounted vertically over a relatively small footprint on the floor. Often such pole stands have wheels for the convenience of the operator in moving them to where they are needed. It is a well known problem in the hospital setting that such wheeled stands are easily unbalanced upon, for example, crossing thresholds or exiting elevators.

A related concern is matter of rapidly mounting and demounting various instruments in a hospital setting. In a crisis situation, it can be important that the proper mounting of an instrument module be accomplished expeditiously. Even in routine operations, a convenient and reliable mounting system would increase efficiency while enhancing patient comfort. In particular, infusion therapy is performed on many patients in the hospital, and due to incompatibilities in drug chemistry or infusion regimen, this often requires several infusion lines. Over the course of a hospital stay, the number of infusion lines, and hence the number of infusion pumps managing the flow within them, is likely to vary. The cost of patient care is reduced if the infusion pumps can rapidly be redeployed where needed.

Examples of infusion pumps that have typically been mounted on pole stands are shown in co-assigned U.S. Patent No. 5,017,192 and U.S. Design Patent No. 278,181. Typically, a single infusion pump has been mounted on the pole stand at one vertical location along the stand. If additional infusion pumps were needed, they would be mounted on separate pole stands, or at a different vertical location along the same pole stand, or on specially designed multi-pole adaptors or stands that provide a plurality of spaced apart vertical pole sections for mounting infusion pumps. Such infusion pumps have been available from Minnesota Mining and Manufacturing Company, St. Paul, Minnesota, under various trade designations, such as the "AVI 200", "AVI 400" and "AVI 480" model series.

Dual channel infusion pumps have also been available in which one pump housing contains two separately controllable infusion pumps. Dual channel infusion pumps have been available from Minnesota Mining and Manufacturing Company, St. Paul, Minnesota, under the trade designations "AVI 840" and "AVI MICRO 845". In the case of dual channel infusion pumps, space on the pole stands is economized by mounting one housing on the pole stand to provide two pumps and two flow channels. Another dual channel infusion pump is available from IMED Corporation, San Diego, California, under the trade designation "GEMINI PC2". Of course, one disadvantage of any dual channel infusion pump is that in effect two infusion pumps are provided even in situations where only one pump is needed.

Specially designed pole stands have been used to support a plurality of infusion pumps. One such pole stand has been sold by Minnesota Mining and Manufacturing Company, St. Paul, Minnesota, under the trade designation "Model 166 Multipump IV Pole". The "Model 166" pole stand has a lower single pole section supporting an upper multiple pole section, which comprises a plurality of spaced apart pole sections. The upper pole sections of that pole stand have been used to support more than one infusion pump at the same vertical level. Specially designed adaptors have also been available to provide more than one spaced apart vertical pole section on a single pole stand. An adaptor of that type has been available from Minnesota Mining and Manufacturing Company under the trade designation "Model 146 IV Pole Adaptor".

U.S. Patent Nos. 4,756,706 and 4,898,578 show a drug infusion system with calculator. That system includes a plurality of infusion pump modules arranged in a vertical stack along with a central management unit, all of which are supported on a single pole stand. The modules are electronically interlinked with the central management unit. U.S. Patent No. 4,356,475 (Neumann et al.) shows a system containing a predetermined number of monitoring devices and at least one central station. That system includes equipment having a bay into which slide-in modules can be introduced in levels one above the other.

The invention provides a system of interconnecting modules, such as medical device modules (e.g., an infusion pump), such that one module (designated the "center" module) can have two modules (designated the "side" modules) connected on opposite sides of the center module but that prevents additional modules from being mounted on the side modules. The modules of the invention are designed to be interchangeable in so far as the system is concerned, i.e., a "side" module of one set-up can be used as a "center" module of another set-up and visa versa. The invention also provides such a system in which the act of attaching the "center" module to a pole stand automatically allows the attachment of "side" modules to the "center" module, which the "center" module would prevent absent its attachment to the pole stand.

Generally, a medical device module of this invention is adapted for use in an interlocking system of similar medical device modules that are adapted to be mounted in side-by-side relationship on a pole stand. The module comprises securing means for securing the module to a pole, and at least two slide connections mounted on opposite sides of said module, with the slide connection on one side of the module being complementary to the slide connection on the other side of the module. Two pair of opposed latching shoulders are provided on the same opposite sides of the module as the two slide connections, with each pair of opposed latching shoulders corresponding to one of the slide connections. Two latching arms are provided. Each latching arm is movably mounted in the module for engaging a pair of opposed latching shoulders on a similar module to enable the module to be mounted on the similar module. The latching arms are movable between an extended position and a withdrawn position. In the extended position, the latching arms extend relative to the slide connections a distance sufficient to be received between a pair of opposed latching shoulders of a similar module when one of the slide connections are mounted on the slide connection of the similar module, and the latching arms block the slide connections from having a slide connection of a similar module mounted thereon. In the withdrawn position, the latching arms are withdrawn relative to the slide connections to allow a slide connection of a similar module to be mounted on the slide connections. Manually operable means is provided for moving the latching arms from their extended position toward their withdrawn position to permit moving one of the latching arms past the opposed latching shoulders of a similar module on which the module is mounted to permit dismounting the module from the similar module.

A second aspect of the invention is a system comprising at least two medical device modules as described above.

Preferably, the manually operable means comprises a push button mounted on each latching arm to facilitate manually pushing the latching arms from their extended position toward their withdrawn positions.

Also, preferably, one of the latching shoulders of each pair of opposed latching shoulders is formed on a structure having a sloped surface along the side facing away from the other latching shoulder. The sloped surface urges the latching arm of a similar module toward its withdrawn position when the slide connection of the similar module is being slidably mounted on the slide connection of the module, until the latching arm of the similar module is received between the pair of opposed latching shoulders. For example, the slide connections may have a direction of slide in the vertical direction, and the sloped surface of the upper shoulder faces generally upwardly. Biasing means is also provided for biasing the latching arms toward their extended position.

Most preferably, automatic means is provided for moving the latching arms against the biasing force of the biasing means to the withdrawn position upon securing the module to a pole via said securing means. Conveniently, each latching arm includes a rack portion having gear teeth. A gear rotatably is mounted in the module, with the gear teeth of the gear in intermeshing relationship with the gear teeth of the rack portions of the latching arms such that rotation of the gear in one direction withdraws the latching arms from their extended position to their withdrawn position. A tongue is provided on one of the latching arms extending into the securing means such that when the tongue is engaged by a pole to which the securing means is secured, the tongue is moved by the pole to move the latching arms to their withdrawn position.

Also, preferably, the slide connections comprise male and female dovetail-type slide connections on opposite sides of the module, with the male and female dovetail-type slide connections being complementary to one another. The dovetail-type slide connections have upper and lower ends relative to the intended use of the module. Each pair of opposed latching shoulders includes an upper latching shoulder and a lower latching shoulder. The lower latching shoulder includes a flange extending generally vertically upwardly from the lower shoulder along an outer edge of the shoulder. The shoulders are generally adjacent the upper ends of the dovetail-type slide connections. Each latching arm has a downwardly-facing notch therein generally adjacent an outer end of the latching arm. The notches of the latching arms are adapted to receive the flanges of similar modules when the latching arms are in their extended position.

Other features will be in part apparent and in part pointed out hereinafter.

The invention will be further described with reference to the drawing wherein corresponding reference characters indicate corresponding parts throughout the several views of the drawing, and wherein:
Figure 1 is a frontal perspective view of three modules of the invention illustrating their being mounted on a single pole stand;
Figure 2 is a top plan view of the three modules of figure 1 mounted on the single pole stand;
Figure 3 is a partial cross-sectional view substantially along line 3-3 of figure 2 illustrating a first condition of the module in which the module is adapted to be mounted on the side of another module;
Figure 4 is a modified cross-sectional view similar to figure 3 illustrating a second condition of the module in which the module is adapted to have other modules set in their first condition mounted on its opposite sides;
Figure 5 is an enlarged exploded diagrammatic top view of complementary slide connections on the modules taken at area 5-5 of figure 2 illustrating details of slide connections that are adapted to help interlock adjacent modules together;
Figure 6 is an enlarged diagrammatic view of a portion 6-6 of two interlocked modules of figure 4 illustrating details of the arrangement when one module is mounted on another;
Figure 7A and 7B are side views of a portion of outer sides the two side modules shown as illustrated in figure 2;
Figure 8 is an enlarged diagrammatic view of selected portions of figure 7A and 7B illustrating various details of the mechanism for preventing mounting of more than one side module on one side of a center module;
Figure 9 is a frontal perspective view of two modules of an alternative embodiment of the invention;
Figure 10 is a cross-sectional view substantially along line 10-10 in figure 9 illustrating details of interlocking slide connections of that embodiment;
Figure 11 is side view of a preferred embodiment of the medical device module of the invention, illustrating a novel interlocking mechanism;
Figure 12 is a front view of a portion of the interlocking mechanism of figure 11 illustrated separately from the medical device module, and showing the extended position of the latching arms;
Figure 13 is a front view similar to figure 12, showing the withdrawn position of the latching arms;
Figure 14 is a top plan view of the interlocking mechanism of figures 11-13, with other portions of the medical device module of figure 11 being shown in phantom, illustrating a tongue mounted on one of the latching arms when it extends into the pole-receiving means and the latching arms in their extended position; and
Figure 15 is a top plan view similar to figure 14, illustrating the position of the tongue when after it is moved by a pole to move the latching arms to their withdrawn position.

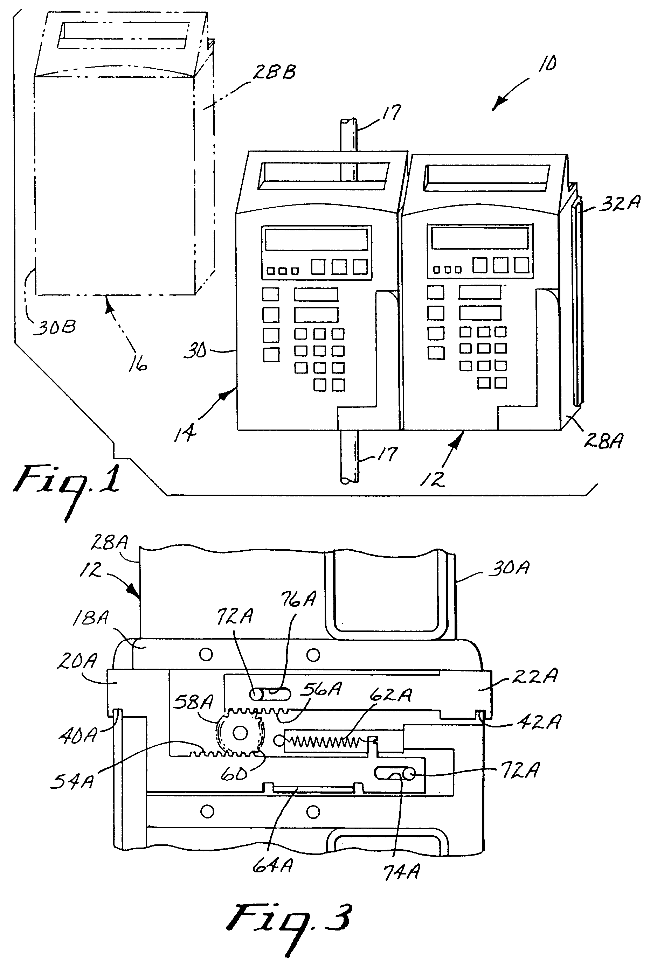

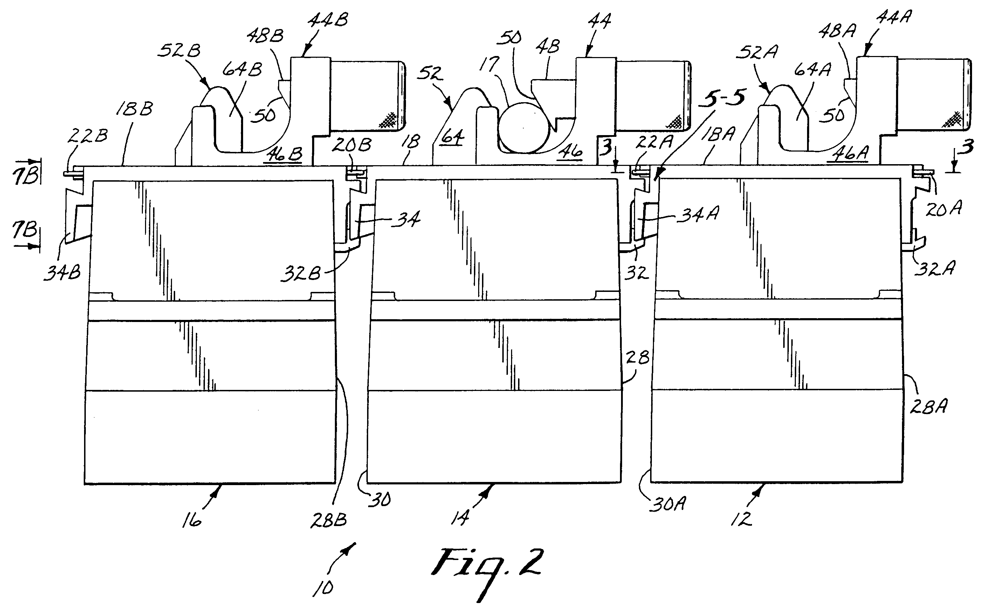

As illustrated in figure 1, the system 10 of the invention comprises a plurality of interchangeable modules 12, 14 and 16 which are adapted to be releasably interlocked in a side-by-side arrangement supported by one of the modules 14 on a pole stand 17. See, also, PCT Publication No. 93/10835.

The modules 12, 14 and 16 may be in the form of three infusion pumps 12, 14 and 16 of the general type described in U.S. Patent Nos. 4,236,880; 4,277,226 4,322,201; 5,017,192; and 5,242,407, as well as European Patent Publication No. 0 510 881. One or more of the modules 12, 14 and 16 may also be of the general type sometimes referred to as "syringe pumps", such as described in U.S. Patent Nos. 4,202,333; 4,298,000; 4,430,079 and 4,597,754. It is also contemplated that the modules 12, 14 and/or 16 of the invention might include other types of modules, such as other medical instrument modules. Examples include patient monitoring equipment, as well as other types of modules that may be employed in medical procedures.

As used herein, the term "interchangeable" refers to the feature of the modules 12, 14 and 16 being capable of being arranged in each other's position. For example, either one of the "side" modules 12 and 16 in figures 1 and 2 could be repositioned in the center or exchanged with each other, and the "center" pump 14 could be positioned at either side. "Interchangeable" is not intended to refer the function of the modules 12, 14 and 16, e.g., one module could be an infusion pump, and another module of the same system could be a patient monitor.

The modules 12, 14 and 16 illustrated in the drawings are substantially identical, and will first be described with reference to the center module 14 and right side module 12. Corresponding features in the "right side" module 12 and "left side" module 16 are indicated by the same reference numerals as the "center" module 14 but are followed by an "A" or "B" respectively. Regardless of which module 12, 14 or 16 is being described, the relevant features of the other modules of the preferred embodiment are identical.

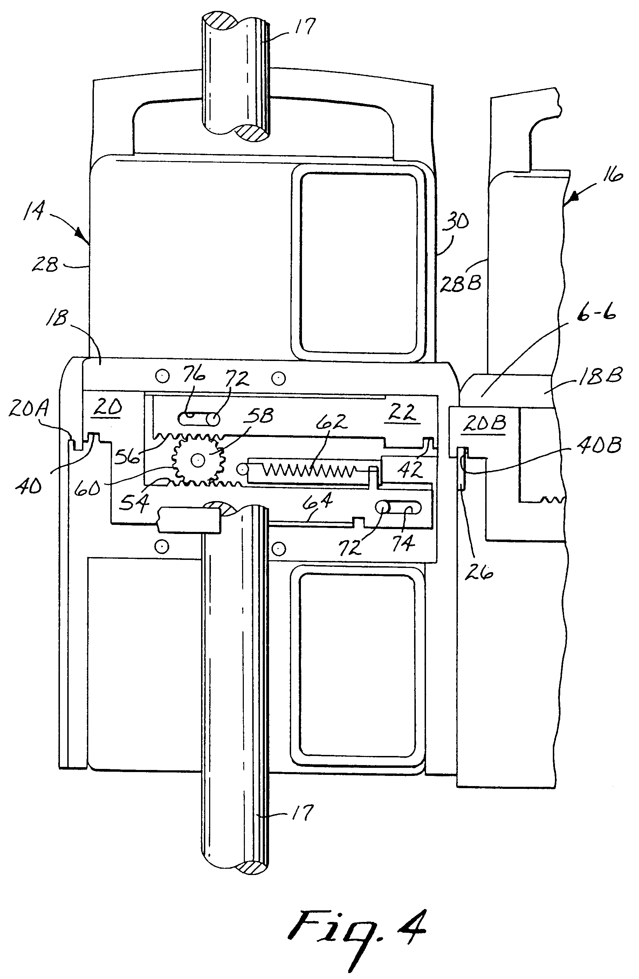

Module 14 generally comprises a frame 18, and interlocking means on the frame 18 for attaching at least two similar modules (e.g., 12 and 16) having interlocking means to the module 14. The interlocking means includes condition-setting means (e.g., latching arms 20 and 22) for setting the interlocking means in a first condition (figure 3) or a second condition (figure 4). As illustrated with "right" and "left" modules 12 and 16 in figures 1-3, in the first condition, the interlocking means is adapted for mounting the module 12 or 16 on a similar module 14. For example, the latching arms 20A and 22A of module 12 are shown in their extended position in figure 3, and one of the latching arms 20B of module 16 is shown in its extended position in figure 4. As illustrated with "center" module 14 in figures 1-2 and 4, in the second condition, the interlocking means is adapted for mounting similar module(s) (e.g., 12 and 14) thereon. For example, the latching arms 20 and 20A of the center module 14 are shown in their retracted position in figure 4.

The arrangement is such that, when the interlocking means of a module (e.g., 12) is set in its first condition, the interlocking means is capable of being mounted on the interlocking means of a similar module (e.g., 14) that has been set in its second condition, but the interlocking means of the module 12 is not capable of having mounted thereon the interlocking means of a similar module (e.g., 16) that has been set in its first condition. The arrangement is also such that, when the interlocking means of a module (e.g., 14) is set in its second condition, it is capable of having mounted thereon the interlocking means of at least two similar modules (e.g., 12 and 14) that have been set in their first condition. Various alternatives as to how this can be accomplished will be described below.

For purposes of illustration, the arrangement in the first condition will be described mostly with respect to "right" module 12, and the arrangement in the second condition will be described mostly with respect to "center" module 14. In the preferred embodiment illustrated in figures 1-8, the difference between the first and second conditions is whether the latching arms 20 and 22 are extended as illustrated at 20A and 22A in figure 3 or retracted as illustrated at 20 and 22 in figure 4.

In the first condition, as shown in figure 3, the latching arms 20A and 22A of module 12 are extended to a first position, or as shown in figure 4 the latching arms 20B of module 16 are extended to the first position, and with either module 12 or 16 the intended result being to latch onto a corresponding latching surface 24 or 26 of the center module 14 (with corresponding latching arms 20A and 22B) to mount module 12 and 16 on the right or left side 28 or 30 of module 14. In the second condition, the latching arms 20 and 22 of module 14 are retracted to a second position (figure 4).

Preferably, at least two slide connections 32 and 34 are provided on opposite sides 28 and 30 of the module 14. The slide connection 32 on one side 28 of each module 14 is complementary to the slide connection 34 on the other side 30 of the module 14. The arrangement is such that the slide connection 32, 32A or 32B on the right side 28 of any module 12, 14 or 16 of one compatible system 10 is complementary to the slide connection 34, 34A or 34B on the left side 30 of any other module 12, 14 or 16 of the same system 10.

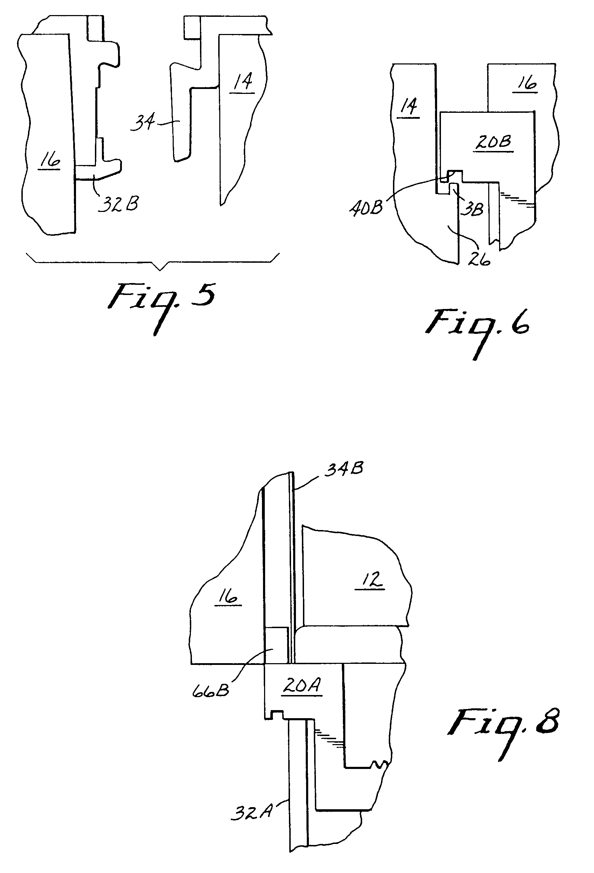

As used herein, the term "complementary" refers to the feature of the slide connections on opposite sides 28 and 30 of the modules being compatible with one another so as to properly fit each other to interchangeably mount the modules 12, 14 and 16 on one another. For example, as illustrated in figures 2 and 5, the slide connections 32, 32A and 32B on the right sides 28, 28A and 28B of the modules 14, 12 and 16, respectively, may comprise female dovetail-type slide connections 32, 32A and 32B, and the slide connections 34, 34A and 34B on the left sides 30, 30A and 30B may comprise "complementary" male dovetail-type slide connections 34, 34A and 34B. The male dovetail-type slide connections 34, 34A and 34B are adapted to be securely engaged in any of the female dovetail-type slide connections 32, 32A and 32B. Figure 5 is a diagrammatic top view of area 5-5 in figure 2 illustrating that the male dovetail-type slide connections 34A are sized to be closely received in the open area of the female dovetail-type slide connections 32.

As illustrated in figures 1, 2 and 7A-B, the slide connections, e.g., 32A, are arranged along the modules 12, 14 and 16 such that the direction of slide in the connection 32, 32A, 32B, 34, 34A and 34B is generally along a vertical axis when the modules 12, 14 and 16 are in use. This orientation of the slide connections 32, 32A, 32B, 34, 34A and 34B facilitates mounting the side modules 12 and 16 on the center module 14. The side modules 12 and 16 merely have to be raised relative to the center module 14, and the lower end of the appropriate slide connection 34A and 32B of the side modules 12 and 16 slide into or around the complementary slide connection 32 and 34, respectively, of the center module 14 to mount the side modules 12 and 16 on the center module 14.

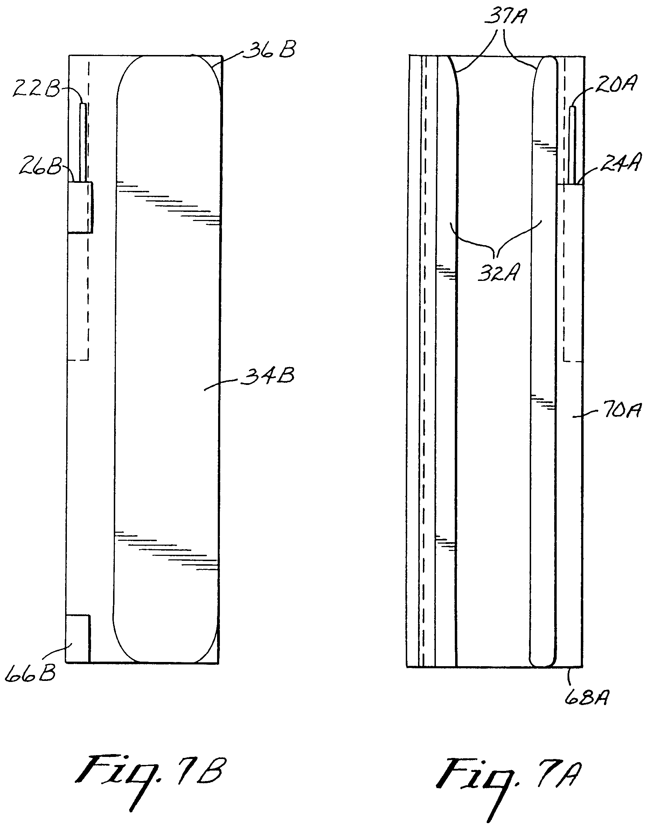

In order to facilitate mounting one module on another, the upper and/or lower ends of the male dovetail-type slide connections 34, 34A and 34B are preferably tapered as illustrated at 36B in figure 7B to ease their introduction into the open slot area of the female-type slide connections 32, 32A and 32B. The open slot area of the female dovetail-type slide connections 32, 32A and 32B may also be flared outwardly adjacent their upper and/or lower ends as illustrated at 37A in figure 7A to guide the male dovetail-type slide connections 34, 34A and 34B into the open slot area of the female slide connections 32, 32A and 32B.

The slide connections 32, 32A, 32B, 34, 34A and 34B preferably extend along a substantial portion of the entire height of the sides 28, 28A, 28B, 30, 30A or 30B of the modules 12, 14 and 16. The height of the slide connections 32, 32A, 32B, 34, 34A and 34B is most preferably at least half of the height of the module 12, 14 or 16. For example, the height of the slide connections 32, 32A, 32B, 34, 34A and 34B may be approximately two thirds of the height of the modules 12, 14 and 16. One example is slide connections 32, 32A, 32B, 34, 34A and 34B having a six inch (150mm) height on modules 12, 14 and 16 having a nine inch (230mm) height.

Preferably, each latching surface (e.g., 26 in figure 6) is formed by a shoulder (also 26) including a flange 38 adapted for engaging a latching arm 20B of a module 16 being mounted thereon. The flange 38 extends generally vertically upwardly from the shoulder 26 along an outer edge of the shoulder 26. As illustrated in figure 6, a notch 40B is provided in each latching arm 20B generally adjacent the outer end of the latching arm 20B. The notches 40B of the latching arms 20B are adapted to receive the corresponding flanges 38 of a center module 14 when the latching arms 20B and 22B are in their first position. (The notch in the opposite latching arm 22 and 22A is designated by the reference numeral 42 or 42A in figures 3 and 4.)

As illustrated in figures 7A and 7B, the shoulders 24 and 26 are generally adjacent the upper ends of the slide connections 32, 32A, 32B, 34, 34A, and 34B. The shoulders 24 and 26 are formed in a generally upwardly-facing direction to support the latching arms 20A or 22A of adjacent modules 12 to mount the adjacent module 12 on the "center" module 14. Alternatively, it is contemplated that the shoulders and latching surfaces (not shown) could be provided adjacent the lower ends of the slide connections.

Each module 12, 14 and 16 has a securing means generally designated 44 (figure 2) for securing the module 12, 14 or 16 to the pole 17. As illustrated in figure 2, the securing means 44 preferably comprises a generally U or J-shaped clamp bracket 46 forming a generally U or J-shaped opening for receiving the pole 17, and a movable clamp jaw member 48 mounted on one side of the bracket 46 for movement to clamp or release a pole 17 between the jaw member 48 and the opposite side of the bracket 46.

The end surface 50 of the jaw member 48 is most preferably inclined at an angle (e.g., 55-60 degrees) relative to the longitudinal axis of movement AX-2 of the jaw member 48 to apply a clamping force to the pole 17 inwardly relative to the J or U-shaped open area defined by the clamp bracket 46. The end surface 50 of the jaw member 48 may be provided with a suitable texture (e.g., a knurled or ribbed surface or rubber coating) to help secure the module 14 on the pole 17. The securing means may alternatively be of the general type shown in U.S. Design Patent No. 278,181.

Automatic means 52 is preferably provided for moving the latching arms 20 and 22 to their second position (figure 4) upon securing the module 14 to a pole 17 via the securing means 44. Most preferably, each latching arm 20 and 22 includes a rack portion 54 and 56 having gear teeth (also at 54 and 56). A gear 58 is rotatably mounted on the frame 18, with the gear teeth 60 of the gear 58 in intermeshing relationship with the gear teeth 54 and 56 of the latching arms 20 and 22. The arrangement is such that rotation of the gear 58 in one direction (counterclockwise in the drawing) withdraws the latching arms 20 and 22 from the first position (figure 4 and latching arm 20A in figure 5) to their second position (latching arms 20 and 22 in figure 5).

Biasing means (e.g., coil spring 62 or 62A) is provided for biasing the latching arms 20 and 22 or 20A and 22A toward their first position (figure 4). The biasing spring 62 or 62A preferably engages the frame 18 at one end of the spring 62 or 62A and one of the latching arms 20 or 20A to bias the latching arms 20, 20A, 20B, 22, 22A and 22B laterally outwardly relative to the module 12, 14 or 16 to their first position.

A tongue 64, 64A is provided on one of the latching arms (left latching arms 20 and 20A in the drawing). As shown in figure 2, the tongue 64, 64A or 64B extends into the securing means 44, 44A and 44B such that when the tongue 64 is engaged by a pole 17 to which the securing means 44 is secured, the tongue 64 is moved by the pole 17 to automatically move the latching arms 20 and 22 to their second position.

As illustrated in figure 8, when the latching arms 20A of one module 12 are extended in their first position, they will engage a blocking member 66B on another module 16 if one attempts to mount another module on a "first" side module 12. The result is that the slide connections of the attempted "second" side module cannot be inserted into the slide connections of the "first" side module 12. In other word, more than one side module is prevented from being mounted on any one side of the center module 14. The arrangement is as illustrated in the drawing where at most two side modules 12 and 16 can be mounted on a center module 14, one side module 12 or 16 on each side of the center module 14. In such an arrangement, the securing means 44 of the center module 14 will have to support at most three modules 12, 14 and 16, and the maximum load will be balanced on opposite sides of the center module 14. The bottom surface 68A on continuous member 70A illustrated in figure 7A constitutes one alternative blocking means for engaging a latching arm to block connection of a "second" side module.

The latching arms 20, 20A, 20B, 22, 22A and 22B are mounted in the frame 18, 18A or 18B for sliding movement on pins 72 and 72A in figures 3 and 4. Slots 74, 74A, 76 and 76A are provided in the latching arms 20, 20A, 20B, 22, 22A and 22B to receive the pins 72, 72A, and to allow movement of the latching arms 20, 20A, 20B, 22, 22A and 22B between their first and second positions.

One possible alternative means for moving the latching arms between their first and second conditions comprises an electrical switch arranged relative to the pole-securing bracket 46 to activate solenoid valves to withdraw the latching arms to their second condition. It is contemplated in that alternative that the solenoid valves would be of the pulse type. Other electromechanical means may also be employed to move the latching arms between their first and second positions.

Another alternative arrangement, which is not illustrated in the drawing, would be to provide downwardly-facing latching surfaces adjacent the lower ends of the slide connections, and upwardly-directed notches in the latching arms, which would also be positioned adjacent the lower end of the slide connection. In that alternative arrangement, the latching arms of a side module would be retracted in the first condition to allow the side module to be mounted on another (center) module in its second condition. The latching arms of the center module would be extended in the second condition to allow the downwardly-facing latching surface of the side module to interlock with the latching arms of the center module. In such an alternative, the latching arms would preferably be biased to their retracted position, and automatically extended to their extended position when the module is mounted on a pole stand.

The latching arms 20, 20A, 20B, 22, 22A and 22B may be considered as latching means for mounting the module 12, 14 or 16 on another module 12, 14 or 16, and the latching surfaces or shoulders 24, 24A, 24B, 26, 26A and 26B may be considered as latch-receiving means for allowing the latching means of another module 12, 14 or 16 to be mounted on the module 12, 14 or 16. One example of a condition-setting means is the tongue 64 which automatically moves the latching means (latching arms 20 and 22) to its second, non-latching position when the module 14 is mounted on the pole 17. In the second, non-latching position, the latching arms 20 and 22 are withdrawn into the module, with the result that the module 12 will allow other modules 14 or 16 to be mounted on its slide connections.

It is also contemplated that a detent (not shown) or positive locking means may be provided to prevent removal of a "side" module from a center module. For example, a detent could be provided on the slide connections, possibly by providing a spring-loaded ball detent in one of the slide connection (e.g., the female dovetail-type slide connections), and a detent-receiving recess in the other slide connection (e.g., the male dovetail-type slide connection) to increase resistance to lifting a side module from the center module. Another example would be to provide a manually operated latch (not shown) to positively lock a side module on the center module. In that arrangement, the side module could not be removed from the center module unless a button or lever was manually operated to release the latch. Such a latch might include a spring bias to its latched position such that the latch of the side module automatically clicks into a latch-receiving recess in the center module to lock them together.

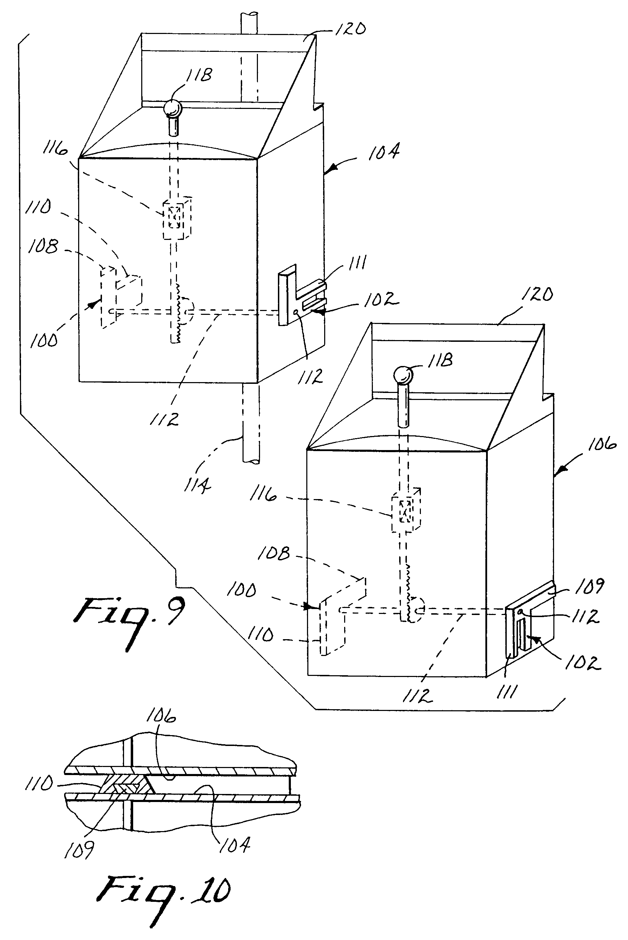

Figures 9 and 10 illustrate an alternative embodiment of the invention in which the interlocking means comprises a pair of generally L-shaped support bodies 100 and 102 rotatably mounted on opposite sides of the module 104 and 106. The same features on modules 104 and 106 are indicated by the same reference character. The modules 104 and 106 shown are "interchangeable" as defined above.

Each of the support bodies 100 and 102 has two legs 108 and 110 or 109 and 111. One leg 108, 109 comprises a male dovetail-type slide connection (also 108, 109) adapted for connection with a complementary female dovetail-type slide connection 110 or 111 of a similar module 106. The other leg 110, 111 comprising a female dovetail-type slide connection (also 110, 111) adapted for connection with a complementary male dovetail-type slide connection 108 or 109 of a similar module 104.

In the embodiment of figures 9 and 10, the condition-setting means comprises means (e.g., axle 112 shown in phantom) for pivotably mounting the support bodies 100 and 102 on opposite sides of the module 104 and 106. The male dovetail-type slide connections 108 and 109 of the support bodies 102 and 104 are aligned in one direction (upwardly in module 104 and in the backward direction in module 106 in figure 9), and the female dovetail-type slide connections 110 and 111 of the support bodies 100 and 102 are aligned in another direction (backwardly in module 104 and downwardly in module 106 in figure 9) generally perpendicular to the direction of the male dovetail-type slide connections 108 and 109.

The support bodies 100 and 102 are pivotable between a first position (module 106 in figure 9) and a second position (module 104 in figure 9). In the first position, one aligned pair of the male or female dovetail-type slide connections 110 and 111 are aligned in the vertical direction. In the second position, the other aligned pair of said male or female dovetail-type slide connections 108 and 109 are aligned in the vertical direction.

The arrangement is such that the first position of the support bodies 100 and 102 corresponds to the first condition of the interlocking means, i.e., that the support bodies 100 and 102 of "side" module 106 are oriented to mount the module 106 on a "center" module 104 but the support bodies 100 and 102 "side" module 106 will not allow a second "side" module (not shown) to be mounted on the "side" module 106. The arrangement is also such that the second position of the support bodies 100 and 102 corresponds to the second condition of the interlocking means, i.e., that the support bodies 100 and 102 of "center" module 104 are oriented to allow a "side" module 106 to be mounted on the "center" module 104.

In the particular embodiment shown in figure 9, the male dovetail-type slide connections 108 and 109 must be aligned in the vertically upward direction ("center" module 104) to allow a "side" module 106 to be mounted thereon. The female dovetail-type slide connections 110 and 111 must be aligned in the vertically downward direction ("side" module 106) to be mounted on the male dovetail-type slide connections 108 or 109 of the "center" module 104. Figure 10 is a cross-sectional illustration of a female dovetail-type slide connection 110 of module 106 mounted on a male dovetail-type slide connection 109 of module 104.

As was the case in the preferred embodiment, means (not shown) is provided for securing the module 104 or 106 on a pole 114. Biasing means generally indicated at 116 in phantom may be provided for biasing the support bodies 100 and 102 to one of their positions. The biasing means 116 shown in figure 9 can either be designed to bias the support bodies 100 and 102 to their first position (module 106) or to their second position (module 104).

The condition-setting means may comprise, in addition to an axle 112, manual or automatic means for setting the module 104 or 106 in its second condition upon securing the module 104 to a pole 114 via the securing means. Although automatic means of the general type described with respect to the embodiment of figures 1-8 is preferred, manual means such as a pull-type knob 118 are shown in figure 9 for manually moving the support bodies 100 and 102 between their first and second positions. The pull-type knob 118 may include a handle (not shown) associated with the handle 120 of the module 104 or 106 to facilitate its operation.

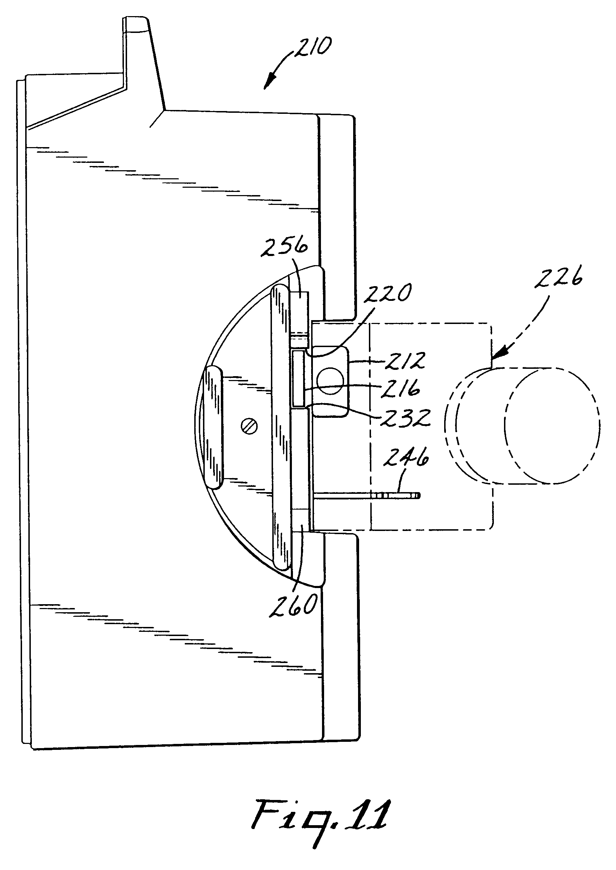

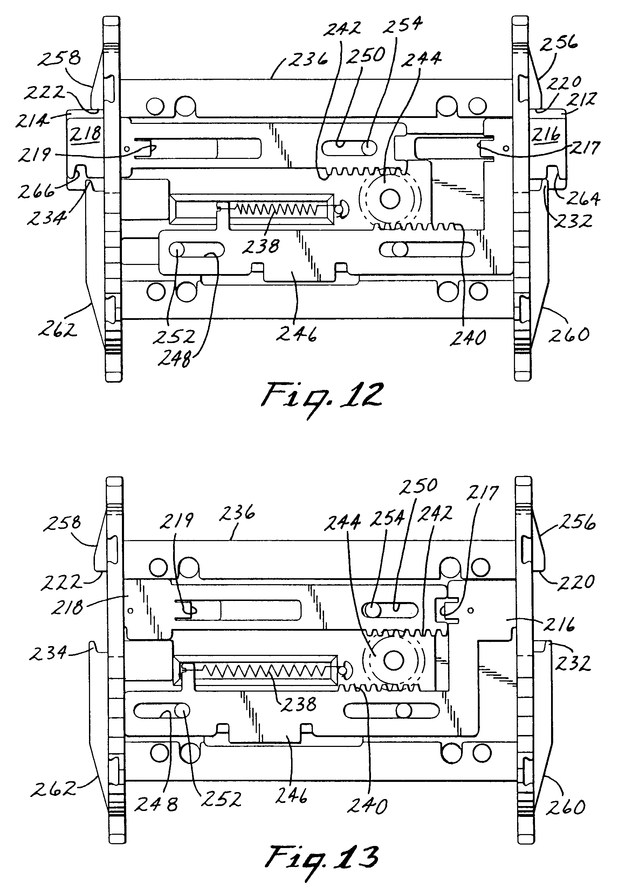

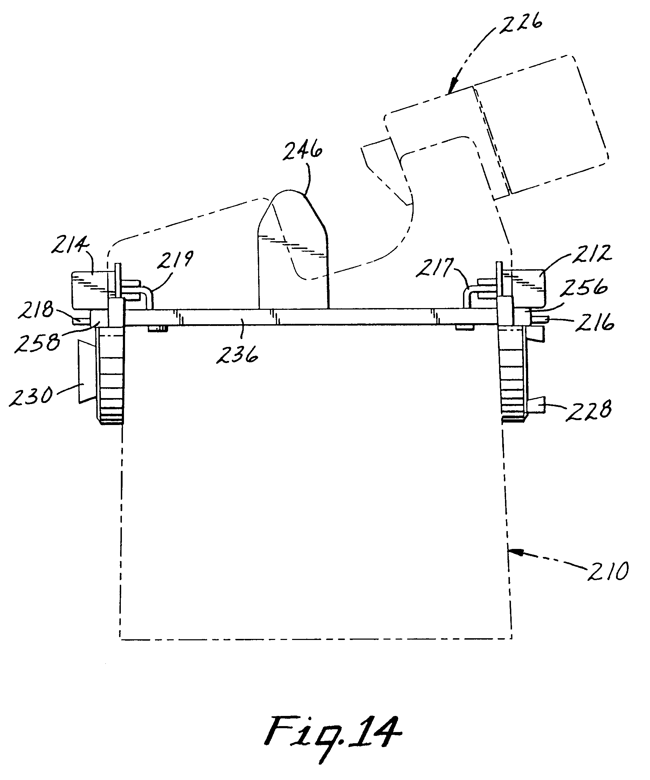

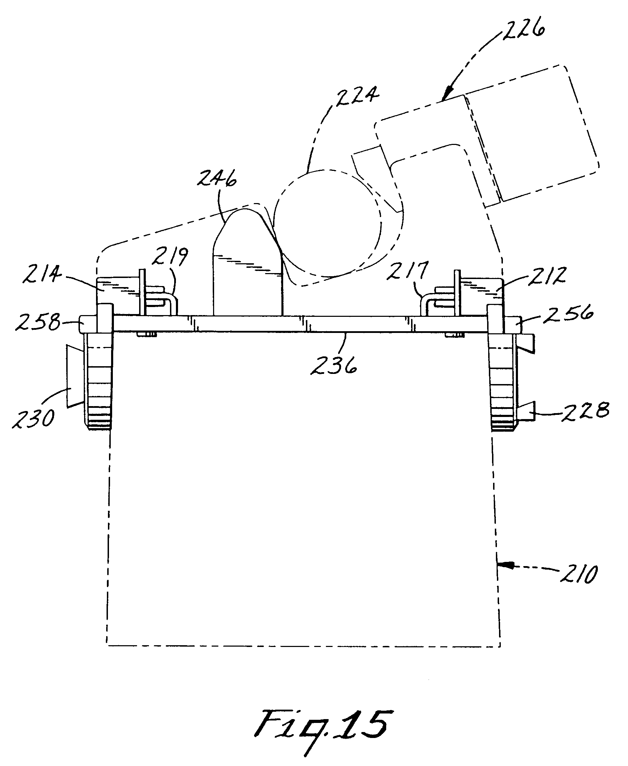

Figures 11-15 illustrate a preferred embodiment of the module, here designated in its entirety by the reference numeral 210. Medical device module 210 is similar in some respects to the medical device module 10 illustrated in figures 1-8, with two particularly notable exceptions being (1) the provision of push buttons 212 and 214 mounted on latching arms 216 and 218 for manually moving the latching arms 216 and 218 to their withdrawn position (figures 13 and 15), and (2) the provision of upper shoulders 220 and 222 which are adapted to engage the upper side of the extended latching arm (not shown) of a similar module mounted on module 210 to prevent lifting that module off until its latching arms are manually moved to their withdrawn position.

More specifically, medical device module 210 is adapted for use in an interlocking system of similar modules (not shown) that are adapted to be mounted in side-by-side relationship on a pole stand (e.g., pole 224 shown in phantom in figure 15). The module 210 generally comprises suitable securing means generally designated 226 for securing the module 210 on a pole 224, and at least two slide connections 228 and 230 mounted on opposite sides of the module 210. The slide connection 228 on one side of the module 210 is complementary to the slide connection 230 on the other side of the module 210.

Two pairs of opposed latching shoulders are provided on the same opposite sides of the module 210 as the two slide connections 228 and 230. For example, upper latching shoulder 220 and lower latching shoulder 232, which are generally adjacent slide connection 228, constitute one pair of opposed latching shoulders 220 and 232 corresponding to slide connection 228. Upper latching shoulder 222 and lower latching shoulder 234, which are generally adjacent slide connection 230, constitute the second pair of opposed latching shoulders 222 and 234, which correspond to slide connection 230.

Each latching arm 216 and 218 is mounted on the frame 236 of the module's interlocking mechanism for movement between an extended position (figures 11, 12 and 14) and a withdrawn position (figures 13 and 15).

In the extended position, both latching arms 216 and 218 extend a distance sufficient to (1) enable one of the latching arms 216 or 218 to be received between a pair of opposed latching shoulders of a similar module when the corresponding slide connection 228 or 230 are mounted on the slide connection of the similar module, thereby enabling the module 210 to be mounted on the similar module; and (2) enable the other of the latching arms 216 or 218 to block the other slide connection 228 or 230 from having a slide connection of a similar module mounted thereon. In other words, the module 210 can be mounted on another module (not shown) when its latching arms 216 and 218 are in their extended position, but no additional modules can be mounted on module 210 when the latching arms 216 and 218 are in their extended position. When the latching arms 216 and 218 are in their extended position, the interlocking mechanism is in its "side" module configuration.

When moved to their withdrawn position, the latching arms 216 and 218 are withdrawn relative to the slide connections 228 and 230 to allow a slide connection of a similar module to be mounted on either or both of the slide connections 228 and 230. When the latching arms 216 and 218 are in their withdrawn position, the interlocking mechanism is in its "center" module configuration.

Manually operable means, such as push buttons 212 and 214, is provided for moving the latching arms 216 and 218 from their extended position (figures 11, 12 and 14) toward their withdrawn position (figures 13 and 15) to permit moving either one of the latching arms 216 or 218 past the opposed latching shoulders of a similar module on which the module 210 is mounted to permit dismounting the module 210 from that similar module (not shown). The arrangement is such that pushing either push button 212 or 214 moves both latching arms 216 and 218 toward the withdrawn position. This allows the exposed push button 212 or 214 on one side of the module 210 to be manually pushed to move the latching arm 218 or 216 on the opposite side of the module 210 toward its withdrawn position to permit dismounting the slide connection 230 or 228 on that opposite side from a slide connection (not shown) on which it was mounted. Each push button 212 and 214 is preferably mounted directly on, or integral with, its corresponding latching arm 216 or 218. For example, each latching arm 216 and 218 may be provided with a button-receiving tab 217 or 219 for mounting the respective push buttons 212 or 214.

Preferably, a suitable biasing means, such as spring 238 is provided for biasing the latching arms 216 and 218 to their extended position (e.g., figure 12), and automatic means is provided for moving the latching arms 216 and 218 against the bias of the biasing means 238 to their withdrawn position (e.g., figure 15) upon securing the module 210 to a pole 224 via the securing means 226.

The automatic means conveniently comprises a rack portion 240 and 242 having gear teeth (also 240 and 242) formed on each latching arm 216 and 218, a gear 244 rotatably mounted on the frame 236 of the interlocking mechanism of the module 210, and a tongue 246 on one of the latching arms 216 extending into the pole-receiving portion of the securing means 226. The gear teeth of the gear 244 are in intermeshing relationship with the gear teeth 240 and 242 of the latching arms 216 and 218 such that rotation of the gear 244 in one direction withdraws both latching arms 216 and 218 from their extended position (figure 12) to their withdrawn position (figure 13), and rotation of the gear 244 in the other direction extends both latching arms 216 and 218 from their withdrawn position to their extended position. The tongue 246 extends into the pole-receiving portion of the securing means 226 such that when the tongue 246 is engaged by a pole 224 to which the securing means 226 is secured, the tongue 246 is moved by the pole 224 to move the latching arms 216 and 218 to their withdrawn position.

Most preferably, each latching arm 216 and 218 is provided with an elongate slot 248 or 250 that slidingly receives a pin 252 or 254 mounted on the frame 236 to prevent movement beyond the extended and withdrawn positions.

Also, preferably, the slide connections 228 and 230 comprise a female dovetail-type slide connection 228 on one side of the module 210, and a male dovetail-type slide connection 230 on the opposite side of the module 210, with the male dovetail-type slide connection 230 being complementary with the female dovetail-type slide connection 228. The slide connections 228 and 230 preferably have a direction of slide in the vertical direction, and the slide connections 228 and 230 have upper and lower ends relative to the intended use of the module 210.

Preferably, at least one latching shoulder 220 or 222 of each pair of latching shoulders is formed on a structure having a sloped surface 256 or 258 along the side of the structure facing away from the other latching shoulder 232 or 234. The sloped surface 256 or 258 is adapted to urge a latching arm of a similar module toward its withdrawn position against the biasing means of that module when the slide connection of the similar module is being mounted on the corresponding slide connection 228 or 230. When the latching arm of the similar module is positioned along the direction of slide between the pair of latching shoulders 220 and 232 or 222 and 234, the biasing means of the similar module biases the latching arm to its extended position so that the latching arm is securely received between the respective latching shoulders 220 and 232 or 222 and 234.

Preferably, the sloped surfaces 256 and 258 are provided along the upper shoulder-forming structure and face generally upwardly to facilitate mounting a "side" module on the "center" module 210 (figures 13 and 15) by urging a latching arm of the "side" module toward its withdrawn position as the corresponding slide connection of the "side" module is slid downwardly along one of the slide connections 228 or 230 of the "center" module 210. Similar sloped surfaces 260 and 262 may be provided on the structures forming the lower latching shoulders 232 and 234. These additional surfaces 260 and 262 face generally downwardly to facilitate mounting a "side" module on a "center" module by urging the latching arms of the "side" module toward their withdrawn position as the "side" module is lifted relative to the "center" module.

Most preferably, the lower latching shoulders 232 and 234 extend outwardly relative to the module 210 slightly farther than the upper latching shoulders 220 and 222. This arrangement is believed to facilitate mounting a "side" module on the "center" module because the latching arm of the "side" module more readily catches such a lower latching shoulder 232 or 234 as a slide connection of the "side" module is lowered into one of the slide connections of the "center" module.

The lower latching shoulders 232 and 234 each preferably include a flange (also 232 or 234) extending generally vertically upwardly from the shoulder 232 or 234 along the outer edge of the shoulder 232 or 234. Each latching arm 216 and 218 has a downwardly-facing notch 264 or 266 generally adjacent the outer end of the latching arm 216 or 218, with the notches 264 and 266 of the latching arms 216 and 218 being complementary with the flanges 232 and 234 such that the notches 264 and 266 are adapted to receive the flanges (similar to flanges 232 and 234) of similar modules when the latching arms 216 and 218 are in their extended position.

The interlocking notch/flange arrangement prevents mounting a second "side" module on the side of a first "side" module. The notch/flange interlock between the flange 232 or 234 of the "center" module and the notch 264 or 266 of the "side" module would maintain the latching arms 216 and 218 of the "side" module in their extended position, and would not allow the latching arms 216 or 218 of the "side" module to retract as one of the latching arms 216 or 218 is engaged by the sloped end surface 256, 258, 260 or 262 of any attempted additional "side" module. The result is that the latching arms of the first "side" module are locked in their extended position, blocking mounting of additional "side" modules on the first "side" module.

The interlocking notch/flange arrangement also prevents a "side" module from being released from a "center" module by merely bumping one of the push buttons 212 or 214. To release a "side" module from the "center" module, the "side" module must be lifted slightly relative to the "center" module and the push button 212 or 214 must be pushed in.

It will be observed that the latching arm of the "side" module cannot be raised past the upper latching shoulder 220 or 222 unless the latching arm is moved toward its withdrawn position, and that the flange 260 or 262 of the "center" module will not allow the latching arm of the "side" module to be so withdrawn unless the "side" module is lifted slightly so that the notch of the latching arm clears the flange 260 or 262 of the "center" module. The distance between the upper end of the flange 260 and 262 and the upper latching shoulder 220 or 222 is preferably just slightly greater than height of the latching arm 216 or 218 to permit sufficient lifting of the "side" module from the "center" module to release the notch of the latching arm from the flange.

This arrangement is believed to help reduce the risk of accidentally releasing the latching arm of a "side" module from the latching shoulders of the "center" module.

The frame 236 of the interlocking mechanism of figures 11-15 is particularly designed to be mounted along the back of the casing of the module 210. The low profile of the mid-portion of the frame 236 minimizes the space requirements of the interlocking mechanism. The interlocking mechanism is particularly designed to be used in medical device modules, such as infusion pumps.

### OPERATION

The operation of the system 10 will be described with reference to the preferred embodiment shown in figures 1-8. The center module 14 is first mounted on a pole stand 17 by clamping the pole 17 with the movable jaw 48. As the securing bracket 46 is brought around the pole 17, the tongue 64 is engaged by the pole 17 and moved out of the open space defined by the securing bracket 46, as illustrated in figure 2 by the relative position of the tongue 64 compared to either tongue 64A or 64B of the "side" modules.

The movement of the tongue 64 by the pole 17 causes the latching arms 20 and 22 to retract into the module 14. This is illustrated by the comparison of figures 3 and 4. In figure 4, the tongue 64 has been engaged by the pole 17 to move latching arm 20 inwardly (rightwardly in figure 4), thus causing the gear 58 to rotate counterclockwise to move the other latching arm 22 inwardly (leftwardly in figure 4). The latching arms 20 and 22 are now withdrawn relative to the latching surfaces 24 and 26 and slide connections 32 and 34 to allow a slide connection of a similar module to be mounted on either/both slide connection 32 and 34. The center module 14 is now ready to have side modules 12 and/or 16 mounted thereon.

To mount the side modules 12 and/or 16 on the center module 14, the slide connections 32A, 32B, 34A or 34B of the side modules 12 or 16 are connected with the adjacent slide connection 32 or 34 of the center module 14 until the latching arm 22A or 20B engages the respective shoulder 24 or 26 of the center module 14. (The latching arms 20A, 20B, 22A and 22B of the "side" modules 12 and 16 are in their first position, in which they extend relative to the latching surfaces and slide connections of the respective side module 12 or 16 a distance sufficient to engage the shoulder 24 or 26 of a similar module 14 when one of the slide connections are mounted on the slide connection of the similar module 14.) At this point, the modules 12, 14 and 16 are properly interconnected on the pole 17.

### Avoidance of Undesired Mounting Arrangements

Mounting of more than one module on one side of a center module 14 may be considered undesirable for a number of reasons, including the possibility of excessively unbalanced loads if two or three modules are mounted on one side of the center module and no modules are mounted on the other side. It may also be desired to limit the total number of modules to be mounted at one location on a pole 17 to prevent the pole-securing means 44 of the center module 14 from being overloaded.

Such undesired arrangements are prevented by the interengagement of the extended latching arms 20A and 22B of the side modules 16 and 12 with the blocking member 66B or blocking surface 68A of the undesired "second" side module. As illustrated in figure 8, if someone were to attempt to mount side module 16 on the right side 28A of a side module 12 that is already mounted on a center module 14, the extended latching arm 20A of the "first" side module 12 would engage the blocking member 66B to prevent the slide connections 32A and 34B from being interconnected.

As various changes could be made in the above constructions without departing from the scope of the invention, it is intended that all matter contained in the above description or shown in the accompanying drawing be interpreted as illustrative and not in a limiting sense.

## Claims

1. An interlocking module system comprising at least two similar medical device modules (210) that are adapted to be mounted in side-by-side relationship on a pole stand (224), each module (210) comprising:
securing means (226) for securing said module (210) to a pole (224);
at least two slide connections (228 and 230) mounted on opposite sides of said module (210), said slide connection (228 or 230) on one side of the module (210) being complementary to said slide connection (230 or 228) on the other side of the module (210);
at least two first shoulders (232 and 234), one first shoulder (232 or 234) being mounted on each opposite side of said module (210) also having a slide connection (228 or 230) mounted thereon; and
two latching arms (216 and 218), each latching arm (216 or 218) being movably mounted in said module (210) for movement between:
an extended position (Figures 12 and 14), wherein said latching arms (216 and 218) block said slide connections (228 and 230) from having a slide connection of yet another module of the system mounted thereon; and
a withdrawn position (Figures 13 and 15), wherein said latching arms (216 and 218) are withdrawn relative to said slide connections (228 and 230) to allow a slide connection of another module of the system to be mounted on said slide connections (228 and 230);
the system being characterized in that:
at least two second shoulders (220 and 222) are provided on each module (210), one second shoulder (220 or 222) being mounted on each side of said module (210) having a first shoulder (232 or 234) mounted thereon so as to form together with the first shoulders (232 and 234) two pair of opposed latching shoulders (220 and 232, or 222 and 234) on the same opposite sides of the module (210) as the two slide connections (228 and 230), each pair of opposed latching shoulders (220 and 232, or 222 and 234) corresponding to one of said slide connections (228 or 230);
when in their extended position (Figures 12 and 14), said latching arms (216 and 218) extend relative to said slide connections (228 and 230) a distance sufficient to be received between a pair of opposed latching shoulders of another module of the system when one of said slide connections (228 or 230) are mounted on the slide connection of the other module to enable said module (210) to be mounted on the other module; and
manually operable means (212 and 214) is provided on each module (210) for moving said latching arms (216 and 218) from their extended position (Figures 12 and 14) toward their withdrawn position (Figures 13 and 15) to permit moving one of said latching arms (216 or 218) past the opposed latching shoulders of another module of the system on which said module (210) is mounted to permit dismounting said module (210) from the other module.

2. A system according to claim 1 further characterized in that the manually operable means (212 and 214) comprises a push button (212 and 214) mounted on each latching arm (216 and 218) to facilitate manually pushing the latching arms (216 and 218) from their extended position (Figures 12 and 14) toward their withdrawn positions (Figures 13 and 15).

3. A system according to claims 1 or 2 further characterized in that one of the latching shoulders (220 or 222) of each pair of opposed latching shoulders (220 and 232, or 222 and 234) is formed on a structure having a sloped surface (256 or 258) along the side facing away from the other latching shoulder (232 or 234) to urge a latching arm of another module of the system toward its withdrawn position when the slide connection of the other module is being slidably mounted on said slide connection (228 or 230) of said module (210) until the latching arm of the other module is received between said pair of opposed latching shoulders (220 and 232, or 222 and 234); the module (210) further comprising biasing means (238) for biasing the latching arms (216 and 218) toward their extended position (Figures 12 and 14).

4. A system according to claim 3 further characterized in that automatic means (240, 242, 244, 246) is provided for moving said latching arms (216 and 218) against the biasing force of the biasing means (238) to the withdrawn position upon securing said module (210) to a pole (224) via said securing means (226).

5. A system according to claims 3 or 4 further characterized in that the slide connections (228 and 230) have a direction of slide in the vertical direction, the sloped surface (256 or 258) facing generally upwardly.

6. A system according to claims 4 or 5 further characterized in that said automatic means comprises:
a rack portion (240 and 242) having gear teeth (240 and 242) on each latching arm (216 and 218);
a gear (244) rotatably mounted in said module (210) and having gear teeth in intermeshing relationship with said gear teeth (240 and 242) of the rack portions (240 and 242) of said latching arms (216 and 218) such that rotation of said gear (244) in one direction withdraws said latching arms (216 and 218) from their extended position (Figures 12 and 14) to their withdrawn position (Figures 13 and 15); and
a tongue (246) on one of said latching arms (216) extending into the securing means (226) such that when said tongue (246) is engaged by a pole (224) to which said securing means (226) is secured, said tongue (246) is moved by the pole (224) to move said latching arms (216 and 218) to their withdrawn position (Figures 13 and 15).

7. A system according to any of claims 3-6 further characterized in that:
said slide connections (228 and 230) comprise male and female dovetail-type slide connections (230 and 228) on opposite sides of said module (210), said male and female dovetail-type slide connections (230 and 228) being complementary to one another, said dovetail-type slide connections (228 and 230) having upper and lower ends relative to the intended use of said module (210);
each pair of opposed latching shoulders (220 and 232, or 222 and 234) including an upper latching shoulder (220 or 222) and a lower latching shoulder (232 or 234), the lower latching shoulder (232 or 234) including a flange (232 or 234) extending generally vertically upwardly from said shoulder (232 or 234) along an outer edge of said shoulder (232 or 234), said shoulders (220, 222, 232, 234) being generally adjacent said upper ends of said dovetail-type slide connections (228 and 230);
each latching arm (216 or 218) having a downwardly-facing notch (264 or 266) therein generally adjacent an outer end of said latching arm (216 or 218), the notches (264 or 266) of said latching arms (216 or 218) being adapted to receive the flanges (232 or 234) of other modules of the system when said latching arms (216 and 218) are in their extended position (Figures 12 and 14).

8. A system according to any of the preceding claims further characterized in that at least one of said medical device modules (210) is an infusion pump (210).
